(19) Europäisches Patentamt
European Patent Office
Office européen des brevets

(11) Numéro de publication: **0 035 925**
**B1**

(12) **FASCICULE DE BREVET EUROPEEN**

(45) Date de publication du fascicule du brevet: **05.12.84**

(51) Int. Cl.$^3$: **C 07 D 401/06,** A 61 K 31/47

(21) Numéro de dépôt: **81400283.8**

(22) Date de dépôt: **24.02.81**

(54) **Nouveaux dérivés de la chloro-3 quinoléine, procédés pour leur préparation, et médicaments les contenant.**

(30) Priorité: **07.03.80 FR 8005152**

(43) Date de publication de la demande:
**16.09.81 Bulletin 81/37**

(45) Mention de la délivrance du brevet:
**05.12.84 Bulletin 84/49**

(84) Etats contractants désignés:
**BE CH DE FR GB IT LI LU NL SE**

(56) Documents cités:
**FR-A-2 354 771**

**Hollemann-Richter, Lehrbuch der organischen Chemie 1960, page 524**

**Le dossier contient des informations techniques présentées postérieurement au dépôt de la demande et ne figurant pas dans le présent fascicule.**

(73) Titulaire: **PHARMUKA LABORATOIRES**
**35 Quai du Moulin de Cage**
**F-92231 Gennevilliers (FR)**

(72) Inventeur: **Champseix, Alain André**
**15, rue Clémenceau**
**F-91406 Orsay (FR)**
Inventeur: **Le Fur, Gérard Roger**
**35, rue du Progrès**
**F-92350 Plessis-Robinson (FR)**

(74) Mandataire: **Gaumont, Robert et al**
**RHONE-POULENC RECHERCHES Service Brevets Pharma 25, Quai Paul Doumer**
**F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 035 925 B1**

Courier Press, Leamington Spa, England.

**Description**

La présente invention a pour objet de nouveaux dérivés de la chloro-3 quinoleine utilisables comme médicaments.

Il est déjà connu par le brevet français FR—A—2354771 des dérivés de la [(quinolyl-4)-3 propyl-1]-4 pipéridine ayant une activité psychotrope et antidépressive.

Les dérivés selon l'invention peuvent être représentés par la formule générale (I)

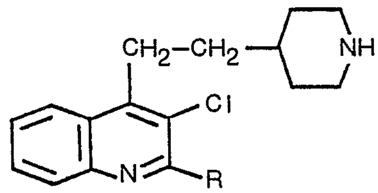

(I)

dans laquelle R représente un atome d'hydrogène, un groupe méthyle ou un groupe phényle.

Les composés de formule générale (I) peuvent être préparés par action du dichlorocarbène $ICCl_2$ sur les dérivés de l'indole de formule (II) et hydrolyse des dérivés N-formylés de formule (III) ainsi obtenus, selon la schéma réactionnel suivant:

(1)

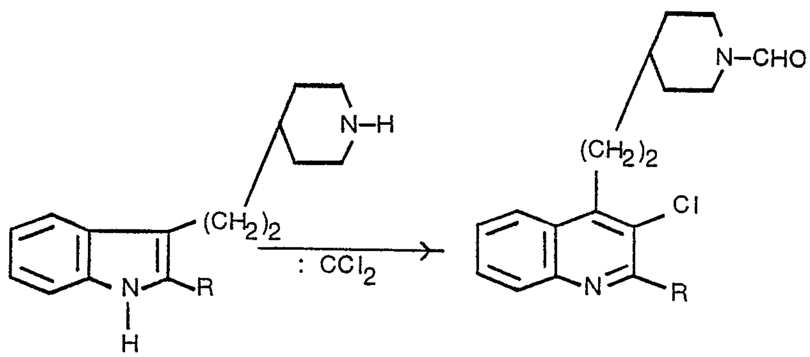


(III)

(II)

(2)

(III) hydrolyse (I)

Dans les formules (II) et (III), R a les mêmes significations que dans la formule (I).

Les dérivés de l'indole de formules (II) sont des composés connus ou que l'on peut préparer par des procédés connus (cf. par exemple: DeGraw et al, J. Heterocyclic Chem. 3 (1), 67—69, 1966; Tacconi et al, Farmaco (Pavia), Ed. Sci. 20(7), 470—481, 1965; Gray et al, Journal of Organic Chemistry, 26, 3368—3372, 1961; brevet anglaise 1 023 781; brevet français de médicament 1693 M; brevet français 2 334 358).

Le dichlorocarbène utilisé dans la réaction (1) est préparé "in situ" par action d'une base sur le chloroforme. La réaction (1) peut être réalisée avantageusement en agitant une solution ou une suspension du dérivé indolique de formule (II) dans le chloroforme avec une solution aqueuse d'hydroxyde de sodium (en particulier une solution aqueuse à 50% d'hydroxyde de sodium), en présence d'un sel d'ammonium quaternaire tel que le chlorure de benzyltriéthylammonium par exemple.

La réaction d'hydrolyse (2) peut être effectuée par chauffage, de préférence à l'ébullition, du composé intermédiaire de formule (III) dans une solution aqueuse d'un acide tel que l'acide chlorhydrique ou l'acide sulfurique.

Les mélanges réactionnels obtenus par les divers procédés décrits précédemment sont traités suivant des méthodes classiques, physiques (évaporation, extraction à l'aide d'un solvant, distillation, cristallisation, chromatographie, etc...) ou chimiques (formation de sel et régénération de la base, etc...) afin d'isoler les composés de formule (I) à l'état pur.

Les composés de formule (I) sous forme de base libre peuvent éventuellement être transformés en sels d'addition avec un acide minéral ou organique par action d'un tel acide au sein d'un solvant approprié.

Les médicaments de la classe des benzodiazépines sont utilisés comme anticonvulsivants, comme hypnotiques et pour le traitement des états d'anxiété et de divers états psychonévrotiques. La présence de récepteurs spécifiques des benzodiazépines dans les membranes de cerveau de rat a été démontrée [SQUIRES et Coll., Nature, *266*, (1977), 732] et le degré d'affinité des benzodiazépines pour ces récepteurs, degré d'affinité mesuré par leur aptitude à déplacer de ses sites de liaison le Diazépam tritié, est en bonne corrélation avec les effets pharmacodynamiques observés chez l'animal et chez l'homme.

Jusqu'a ce jour, en dehors des benzodiazépines et des composés du document FR—A—2354771, aucun médicament agissant par ailleurs sur le système nerveux central ne s'est montré capable de déplacer, de manière significative, le Diazépam de ses sites de liaison [cf. BRAESTRUP et Coll., Europ. J. Pharmacol. 48, (1978) 26].

Les produits de l'invention, bien que de structure différente de celles des benzodiazépines, déplacent le Diazépam de ses sites de liaison. Ils peuvent donc trouver des applications comme hypnotiques, comme anticonvulsivants, et dans le traitement des états de tension et d'anxiété résultant de circonstances "stressantes" ou de troubles somatiques liés à des facteurs émotionnels.

Ils sont utilisables pour le traitement des états psychonévrotiques se manifestant pour des symptômes d'anxiété, d'appréhension, de fatigue, d'agitation ou de dépression.

Les exemples suivants illustrent l'invention. Les données relatives aux spectres de résonance magnétique nucléaire (en abrégé: spectres R.M.N.) figurant dans ces exemples concernent la résonance magnétique nucléaire des protons des composés à l'état de base. Pour effectuer les mesures de résonance magnétique nucléaire, les composés sont mis en solution dans le chloroforme deuteré. La référence utilisée est le tétraméthylsilane.

Exemple 1

Chloro-3 [(pipéridyl-4)-2 éthyl]-4 quinoléine

A une suspension bien agitée de 45,8 g d'[(indolyl-3)-2-éthyl]-4 pipéridine et 1 g de chlorure de benzyltriethylammonium dans 500 ml de chloroforme exempt d'éthanol, on ajoute lentement, en deux heures, en maintenant à la température ambiante (environ 20°C), une solution de 60 g d'hydroxyde de sodium dans 120 ml d'eau. La dissolution est progressive. Après 72 heures à température ambiante, on sépare les deux phases par décantation. La phase aqueuse est extraite par deux fois 100 ml de chloroforme. Les phases organiques sont rassemblées et concentrées par élimination du chloroforme.

Le résidu obtenu est chauffé au reflux pendant une heure dans 400 ml d'une solution aqueuse 5 N d'acide chlorhydrique. Après refroidissement à 0°C, on alcalinise au moyen de 250 ml d'une solution aqueuse 10 N d'hydroxyde de sodium, puis on extrait par 300 ml de chloroforme. La phase organique est lavée par 500 ml d'eau, séchée sur sulfate de magnésium, puis concentrée par élimination du chloroforme. On obtient ainsi 61,2 g d'un produit huileux. Ce produit est fixé sur colonne de silice, puis on élue par un mélange chloroforme-diéthylamine

$$\frac{90}{10}.$$

On obtient 25 g de chloro-3 [(pipéridyl-4)-2 éthyl]-4 quinoléine, dont le chlorohydrate fond à 190°C.
Spectre R.M.N. du produit obtenu:

Les déplacements chimiques δ des protons sont les suivants:

| | | | |
|---|---|---|---|
| —aromatiques | δ | : | 7,3—8,8 ppm |
| —$CH_2$—N et $CH_2$—Ar | δ | : | 2,3—3,4 ppm |

Exemple 2

Méthyl-2 chloro-3 [(pipéridyl-4)-2 éthyl]-4 quinoléine

On opère comme à l'exemple 1, en partant de 5,9 g de [(méthyl-2 indolyl-3)-2 éthyl]-4 pipéridine et 0,12 g de chlorure de triéthylbenzylammonium dans 60 ml de chloroforme et de 7,5 g d'hydroxyde de sodium dans 15 ml d'eau. On obtient finalement 2,7 g de méthyl-2 chloro-3 [(pipéridyl-4)-2 éthyl]-4 quinoléine sous forme de monochlorhydrate qui fond à 255°C.
Spectre R.M.N. du produit obtenu:

| | | | |
|---|---|---|---|
| —aromatiques | δ | : | 7,4—8,2 ppm |
| —$CH_3$Ar | δ | : | 2,8 ppm |
| —$CH_2$N et $CH_2$Ar | δ | : | 2,4—3,4 ppm |

Exemple 3

Phényl-2 chloro-3 [(pipéridyl-4)-2 éthyl]-4-quinoléine

On opère comme à l'exemple 1, en partant de 12 g de [(phényl-2 indolyl-3)-2 éthyl]-4 pipéridine et 0,2 g de chlorure de triéthylbenzylammonium dans 110 ml de chloroforme et de 12 g d'hydroxyde de sodium dans 24 ml d'eau. On obtient finalement 1,05 g de phényl-2 chloro-3 [(pipéridyl-4)-2 éthyl]-4 quinoléine sous forme de chlorhydrate fondant à 233°C.

Spectre R.M.N. du produit obtenu:

—aromatiques $\delta$ : 7,2—8,4 ppm

—$CH_2N$ et $CH_2Ar$ $\delta$ : 2,2—3,4 ppm

La [(phényl-2 indolyl-3)-2 éthyl]-4 pipéridine, produit de départ, a été préparée de la manière suivante:

Une suspension bien agitée contenant 39,5 g de [(phényle-2 indolyl-3)-2 éthyl]-4 pyridine ensolution dans 40 ml d'acide acétique et 2 g d'oxyde de platine Adams est maintenue, à la température ambiante sous une pression d'hydrogène correspondant à une surpression de 50 mm d'eau par rapport à la pression atmosphérique, jusqu'à cessation de l'absorption de gaz.

Le platine est ensuité séparé par filtration et la solution acétique est concentrée par évaporation. Le résidu huileux obtenu est alcalinisé par addition d'une solution aqueuse 11 N d'hydroxyde de sodium et on extrait par 500 ml de chloroforme. La phase chloroformique est séchée sur sulfate de magnésium, puis concentrée par élimination du chloroforme. On obtient ainsi 37,8 g de [(phényl-2 indolyl-3)-2 éthyl]-4 pipéridine qui fond à 183°C.

Spectre R.M.N du produit obtenu:

—aromatiques $\delta$ : 7,2—7,8 ppm

—$CH_2Ar$ et $CH_2N$ $\delta$ : 2,4—3,3 ppm

La [(phényl-2 indolyl-3)-2 éthyl]-4 pyridine a été préparée selon la méthode de P. BRUNI, Ann. Chim. (ROME) 1967, 57 (4), 376—81.

PROPRIETES PHARMACOLOGIQUES

AFFINITE POUR LES SITES RECEPTEURS CEREBRAUX DES BENZODIAZEPINES

Cette affinité est mesurée par l'aptitudes des produits à déplacer le Diazépam tritié ($^3H$ Diazépam) de son site de liaison et est exprimée par une valeur $K_i$, en micromoles ($\mu M$), qui est calculée par la formule:

$$K_1 = \frac{IC_{50}}{1 + \frac{C}{K_D}}$$

dans laquelle C représente la concentration de $^3H$ Diazépam, $K_D$ une constante d'affinité égale à 2,74 $\mu M$ et $IC_{50}$ la concentration nécessaire pour obtenir une inhibition de 50% de la liaison du $^3H$ Diazépam.

Les produits ont été testés selon la protocole de MOHLER et coll., Life Science, 1977, *20*, 2101.

On a obtenu les résultats suivants:

| Produits | $K_i$ ($\mu M$) |
|---|---|
| Exemple 1 | 2 |
| 2 | 1 |
| 3 | 0,5 |

PROPRIETES TOXICOLOGIQUES

Les toxicités aigües des composés selon l'invention ont été déterminées chez la souris mâle $CD_1$ (Charles RIVER) par voie orale. Les $DL_{50}$ ont été calculés, après 3 jours d'observation, par la méthode cumulative de J. J. REED et H. MUENCH (Amer. J. Hyg. 1938, *27*, 493).

Les composés selon l'invention se comportent comme des substances relativement peu toxiques chez la souris, puisque les $DL_{50}$ des composés se situent entre 200 et 1000 mg/kg.

UTILISATION THERAPEUTIQUE

Les composés de l'invention et leurs sels pharmaceutiquement acceptables peuvent être utilisés en thérapeutique humaine, sous forme de comprimés, capsules, gélules, suppositoires, solutions ingérables ou injectables, etc... comme hypnotiques, anticonvulsivants et pour le traitement des états d'anxiété et de divers états psychonévrotiques.

La posologie dépend des effets recherchés et de la voie d'administration utilisée. Par exemple, par voie orale, elle peut être comprise entre 5 et 250 mg de substance active par jour, avec des doses unitaires allant de 1 à 50 mg.

## Revendications

1. Composés de formule générale (I)

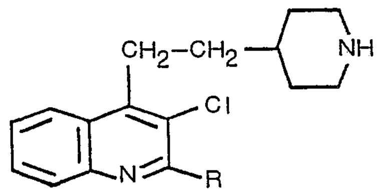

(I)

dans laquelle R représente un atome d'hydrogène ou un groupe méthyle ou phényle, et leurs sels d'addition avec les acides minéreaux ou organiques.

2. Composés selon la revendication 1 dans lesquels R est un groupe méthyle ou phényle.

3. Composé selon la revendication 2 de formule:

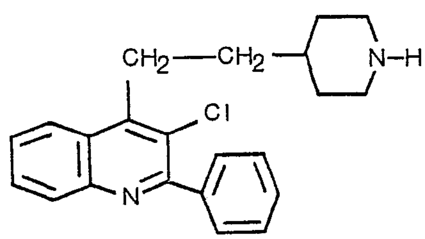

et ses sels d'addition avec les acides minéraux ou organiques.

4. Procédé de préparation des composés selon la revendication 1, caractérisé en ce que l'on fait réagir le dichlorocarbène $ICCl_2$ sur un dérivé de l'indole de formule (II)

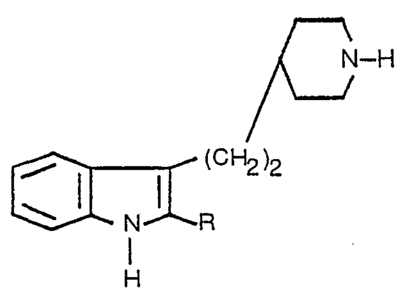

(II)

et hydrolyse le composé de formule (III)

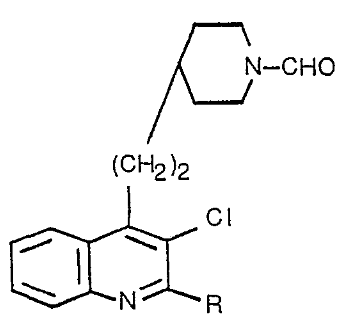

(III)

ainsi obtenu, R ayant dans les formules (II) et (III) les mêmes significations que dans la formule (I) de la revendication 1.

5. Médicament utile comme hypnotique, anticonvulsivant et pour le traitement des états d'anxiété et des états psychonévrotiques, caractérisé en ce qu'il contient, en tant que principe actif, un composé répondant à la formule (I) de la revendication 1 ou un sel d'un tel composé avec un acide pharmaceutiquement acceptable.

6. Médicament selon la revendication 5, caractérisé en ce qu'il contient, comme principe actif, le composé de formule:

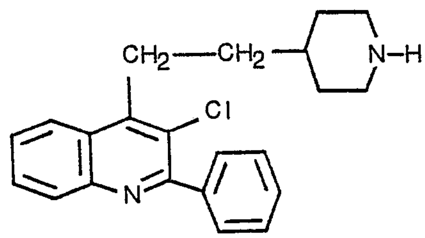

ou un sel de ce composé avec un acide pharmaceutiquement acceptable.

**Claims**

1. Compounds of the general formula (I)

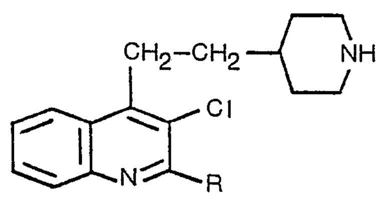

(I)

in which R represents a hydrogen atom or a methyl or phenyl group, and their addition salts with inorganic or organic acids.

2. Compounds according to Claim 1 in which R is a methyl or phenyl group.

3. Compound according to Claim 2, of the formula

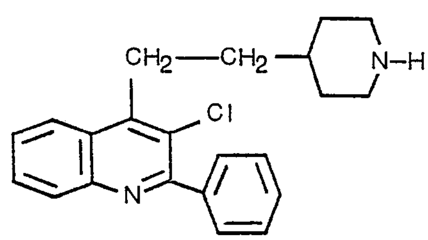

and its addition salts with inorganic or organic acids.

4. Process for the preparation of the compounds according to Claim 1, characterised in that dichlorocarbene $ICCl_2$ is reacted with an indole derivative of the formula (II)

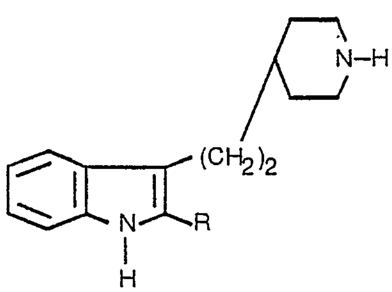

(II)

and the compound of the formula (III)

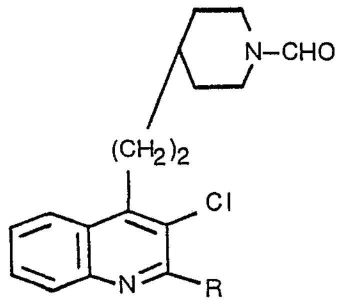

(III)

thus obtained is hydrolysed, R in the formulae (II) and (III) having the same meanings as in formula (I) of Claim 1.

5. A medicament useful as a hypnotic and anticonvulsant and for the treatment of anxiety conditions and psychoneurotic conditions, characterised in that it contains, as the active principle, a compound corresponding to formula (I) of Claim 1 or a salt of such a compound with a pharmaceutically acceptable acid.

6. Medicament according to Claim 5, characterised in that it contains, as the active principle, a compound of the formula

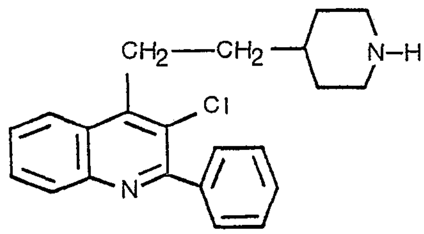

or a salt of this compound with a pharmaceutically acceptable acid.

**Patentansprüche**

1. Verbindungen der allgemeinen Formel (I)

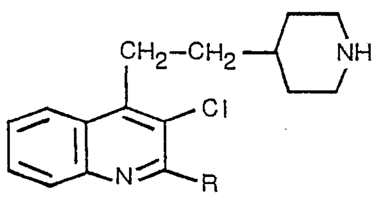

(I)

worin R ein Wasserstoffatom oder eine Methyl- oder Phenylgruppe bedeutet und ihre Additionssalze mit Mineralsäuren oder organischen Säuren.

2. Verbindungen gemäß Anspruch 1, worin R eine Methyl- oder Phenylgruppe ist.

3. Verbindung gemäß Anspruch 2 der Formel:

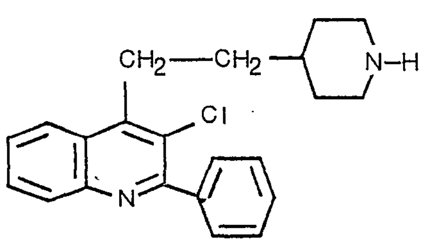

und ihre Additionssalze mit organischen oder Mineralsäuren.

4. Verfahren zur Herstellung von Verbindungen gemäß Anspruch 1, dadurch gekennzeichnet, daß man Dichlorcarben $CCl_2$ auf ein Derivat des Indols der Formel (II)

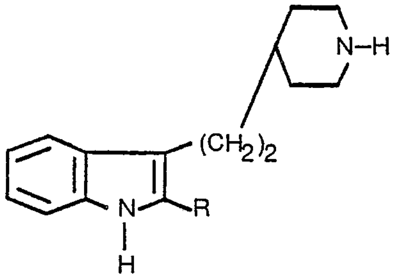

(II)

einwirken läßt und die so erhaltene Verbindung der Formel (III)

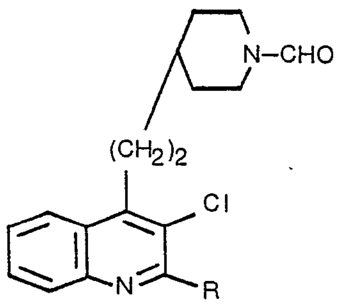

(III)

worin R in den Formeln (II) und (III) dieselben Bedeutungen hat wie in Formel (I) von Anspruch 1, hydrolysiert.

5. Arzneimittel, das als Hypnotikum, Antikonvulsivum und zur Behandlung von Angstzuständen und pyschoneurotischen Zuständen nützlich ist, dadurch gekennzeichnet, daß es als aktives Prinzip eine Verbindung entsprechend der Formel (I) von Anspruch 1 oder ein Salz einer solchen Verbindung mit einer pharmazeutisch annehmbaren Säure enthält.

6. Arzneimittel gemäß Anspruch 5, dadurch gekennzeichnet, daß es als aktives Prinzip die Verbindung der Formel

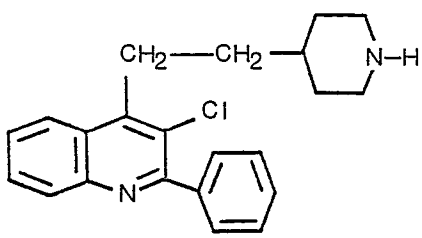

oder ein Salz dieser Verbindung mit einer pharmazeutisch annehmbaren Säure enthält.